# NOUVEAU FASCICULE DE BREVET EUROPEEN

(11) **EP 1 411 895 B2**
(45) Date de publication et mention de la décision concernant l'opposition: **29.07.2009**
(45) Mention de la délivrance du brevet: 17.08.2005
(21) Numéro de dépôt: 02791502.4
(22) Date de dépôt: 18.07.2002
(51) Int. Cl.: A61K 9/107, A61K 31/366

(54) **COMPOSITION PHARMACEUTIQUE A USAGE ORAL COMPRENANT UN PRINCIPE ACTIF SUSCEPTIBLE DE SUBIR UN IMPORTANT EFFET DE PREMIER PASSAGE INTESTINAL**
ORALE PHARMAZEUTISCHE ZUSAMMENSETZUNG MIT EINEM WIRKSTOFF, DER BEI SEINER ERSTEN DARMPASSAGE STARKEN WIRKUNGEN AUSGESETZT WERDEN KANN
ORAL PHARMACEUTICAL COMPOSITION COMPRISING AN ACTIVE PRINCIPLE CAPABLE OF BEING SUBJECTED TO HIGH EFFECT DURING ITS FIRST PASSAGE THROUGH THE INTESTINE

(30) Priorité: 27.07.2001 FR 0110094
(43) Date de publication de la demande: 28.04.2004
(73) Titulaire: GATTEFOSSE HOLDING, 69800 St Priest (FR)
(72) Inventeur: BENAMEUR, Hassan, F-68140 Munster (FR); JANNIN, Vincent, F-69007 Lyon (FR); ROULOT, Delphine, F-69440 Riverie (FR)
(74) Mandataire: Vuillermoz, Bruno
(86) Numéro de dépôt international: PCT/FR2002/002566
(87) Numéro de publication internationale: WO 2003/011207

(56) Documents cités:
- WO-A-00/37057
- WO-A-00/50007
- WO-A-95/08983
- WO-A-02/053131

## Description

L'invention concerne une nouvelle composition à usage oral comprenant un principe actif susceptible de subir un important effet de premier passage intestinal. En tant que principes actifs sont plus particulièrement illustrées les statines et notamment la simvastatine.

On sait de par les documents Drug Metab. Disp. (1995) 23 : 279-84 et Transplantation (1992) 53 : 596-602 que le métabolisme intestinal joue un rôle important dans la biodisponibilité d'un certain nombre de principes actifs. Aussi, des études ont montré que la biodisponibilité d'un principe actif pouvait être améliorée en bloquant ou en réduisant le métabolisme intestinal (voir Clin. Pharmacol. Ther. (1997) 61 : 401-9) plutôt que d'agir sur le métabolisme hépatique. La solution consistant à bloquer le métabolisme intestinal présente un certain risque puisqu'elle agit directement sur le système de régulation. En effet, les transporteurs cellulaires ont une fonction bien déterminée dont la régulation dépend de la concentration luminale en ligands. Si par exemple, la concentration en ligand diminue, le nombre de transporteurs augmente.

Le Demandeur a donc cherché à réduire le métabolisme intestinal que subissent un certain nombre de molécules.

Bien que tous les principes actifs susceptibles de subir un premier effet de passage intestinal soient concernés par l'invention, cette dernière est plus particulièrement décrite en relation avec les statines et notamment la simvastatine sans pour autant que ce ne soit limitatif.

Les statines constituent une famille thérapeutique qui agit en inhibant l'hydroxyméthylglutaryl (HMG) Coenzyme A-réductase, enzyme limitant la synthèse hépatique du cholestérol et stimulant l'activité des récepteurs LDL (Low Density Lipoprotein). De part ce mécanisme d'action, les statines sont essentiellement utilisées en tant qu'agent hypocholestérolémiant. Un certain nombre d'études ont par ailleurs démontré que les statines avaient un effet préventif sur les maladies cardio-vasculaires, de même qu'elles entraînaient une régression de la plaque d'athérome. Il existe actuellement six statines, respectivement la lovastatine, la pravastatine, la fluvastatine, l'atorvastatine, la cérivastatine et enfin la simvastatine, plus particulièrement illustrées dans la suite de la description.

La simvastatine est un pro-médicament obtenu par synthèse à partir du produit de fermentation d'*aspergillus terreus*. Cette molécule, bien connue, de formule brute C₂₅H₃₈O₅, est une lactone, dont l'activité est déclenchée grâce à l'ouverture par réaction enzymatique ou chimique de sa fonction lactone. En pratique, la simvastatine est rendue active par hydroxylation en β-hydroxyacide. Physiquement, la simvastatine se présente sous forme d'une poudre cristalline blanche pratiquement insoluble dans l'eau mais très soluble dans le chloroforme, le méthanol et l'éthanol. Après administration orale, la simvastatine est hydrolysée après absorption intestinale, et hépatique, en sa forme béta-hydroxy-acide, son principal métabolite, lequel est à l'origine de l'effet inhibiteur compétitif et réversible sur l'HMG CoA-réductase.

Si la simvastatine est bien résorbée dans le tractus gastro-intestinal (à raison de 90 %), elle subit en revanche un puissant effet de premier passage hépatique, en particulier par le cytochrome CYP 3A4. Ce phénomène est notamment décrit dans le document Clin. Pharmacokinet 24(3) : 195-202, 1993, lequel indique que la biodisponibilité systémique de la simvastatine est de seulement 7 % de la dose ingérée. Une solution permettant d'améliorer la biodisponibilité de la simvastatine est d'inhiber l'action du cytochrome CYP 3A4 par des agents inhibiteurs tels que l'itraconazole, le détocoriazole ou encore le jus de pamplemousse. Cependant, cette solution n'est pas satisfaisante dans la mesure où elle peut conduire, comme déjà dit, à une dérégulation du métabolisme. Le Demandeur a, en outre, constaté que la simvastatine subissait un fort effet de premier passage intestinal, problème qui n'était a priori pas connu auparavant pour cette molécule.

Dès lors, le problème que se propose de résoudre l'invention est d'améliorer la biodisponibilité systémique des principes actifs susceptibles de subir un fort effet de premier passage intestinal en diminuant au maximum, et non en bloquant, cet effet. En conséquence, l'effet de premier passage hépatique, dans l'hypothèse où il existe, sera largement diminué dans la mesure où plus de molécules seront disponibles au niveau du foie.

Pour ce faire, l'invention concerne l'utilisation d'un système auto-micro-émulsionable au contact d'une phase aqueuse, ledit système comprenant :
- une quantité thérapeutiquement efficace d'un principe actif susceptible de subir un effet de premier passage intestinal;
- une phase lipophile comprenant un mélange de mono, di, et triesters de glycérol et de mono et diesters de PEG avec au moins un acide gras choisi dans le groupe comprenant les acides gras en C₈-C₁₈ ;
- une phase tensioactive comprenant un mélange de mono, di et triesters de glycérol et de mono et diesters de PEG avec les acides caprylique (C₈) et caprique (C₁₀) ;
- une phase cotensioactive comprenant au moins un ester d'alcool avec au moins un acide gras choisi dans le groupe comprenant les esters caprylique du propylène glycol, les esters laurique du propylène glycol et les esters oléïque du polyglycérol.
- le ratio TA/CoTA étant compris entre 0,2 et 6,
pour la préparation d'une composition pharmaceutique à usage oral destinée à diminuer le métabolisme intestinal du principe actif.

L'invention concerne aussi une composition pharmaceutique à usage oral sous forme d'un système auto-micro-émulsionable au contact d'une phase aqueuse, ledit système comprenant :
- une quantité thérapeutiquement efficace d'un principe actif susceptible de subir un effet de premier passage intestinal;
- une phase lipophile comprenant un mélange de mono, di, et triesters de glycérol et de mono et diesters de PEG avec au moins un acide gras choisi dans le groupe comprenant les acides gras en C₈-C₁₈ ;
- une phase tensioactive comprenant un mélange de mono, di et triesters de glycérol et de mono et diesters de PEG avec les acides caprylique (C₈) et caprique (C₁₀) ;
- une phase cotensioactive comprenant au moins un ester d'alcool avec au moins un acide gras choisi dans le groupe comprenant les esters capryliques du propylène glycol,
- le ratio TA/CoTA étant compris entre 0,2 et 6,
à l'exclusion de la formule constituée de 1,4% d'isotrétinoïne, de 9,7% de Labrasol®, de 13,9% de Capryol®, de 75% de Gelucire® 44/14, ainsi que de la formule constituée de 5% de progestérone, de 8,5% de Labrasol®, de 65% de Gelucire®, de 17,5% de Capryol® PGMC, de 2% de Labrafil® M1944, et de 2% de Labrafac® CC.

Les systèmes auto-micro-émulsionables, dont il est question dans l'invention, sont connus sous la dénomination SMEDDS®, marque déposée par le Demandeur signifiant Self Micro Emulsifying Drug Delivery System, et plus particulièrement décrits dans les documents EP-A-670 715 et US-A-6 054 136 correspondant. D'autres systèmes sont décrits dans WO 00 37 057, ainsi que dans WO 02 093131 publié après la date de priorité du présent brevet. Le Demandeur a constaté que de manière tout aussi surprenante qu'inattendue, les constituants du SMEDDS® permettaient d'augmenter la vitesse de solubilisation des principes actifs de sorte à saturer rapidement les sites enzymatiques responsables de l'effet de premier passage intestinal présents au niveau des villosités, ayant ainsi pour effet de rendre disponible très vite le principe actif en excès et donc d'augmenter la vitesse d'absorption. Le SMEDDS® permet donc de diminuer le métabolisme intestinal et donc d'augmenter la biodisponibilité du principe actif.

Dans les documents précités, il est indiqué que de par la formation de la micro-émulsion au contact d'une phase aqueuse, les SMEDDS® permettent de solubiliser les principes actifs insolubles dans l'eau, et par conséquent, d'augmenter la biodisponibilité du ou des actifs micro-émulsionnés qu'ils véhiculent. Plus précisément, le SMEDDS® permet de solubiliser le principe actif insoluble en le présentant sous forme d'une structure supramoléculaire multiparticulaire. Les documents précités ne décrivent donc que le problème de la solubilité des actifs, laquelle est améliorée par la formulation SMEDDS®. Ils s'ensuit que plus la solubilité augmente et plus la biodisponibilité est améliorée.

Cependant, il n'est nullement fait référence, dans ces documents, ni à la propriété des SMEDDS® d'augmenter la vitesse de solubilisation des principes actifs, ni à l'effet sur la vitesse d'absorption au niveau intestinal qui se trouve améliorée. Aussi, le Demandeur a constaté que de manière tout à fait surprenante, l'incorporation d'un principe actif présentant un puissant effet de premier passage intestinal dans un système auto-micro-émulsionable permettait de diminuer l'effet de premier passage intestinal, et donc d'améliorer la biodisponibilité systémique de la molécule active. Comme déjà dit, l'utilisation du SMEDDS® augmente la vitesse de solubilisation de sorte que les sites enzymatiques intestinaux sont rapidement saturés et que le surplus des principes actifs est rendu immédiatement disponible dans la circulation sanguine. Il s'ensuit que la vitesse d'absorption est augmentée et que la disponibilité de l'actif au niveau hépatique est plus grande et le passage systémique est donc plus important proportionnellement. On n'agit donc pas en aval (au niveau du foie) mais en amont (au niveau de l'intestin).

Les SMEDDS® peuvent se présenter, à température ambiante, sous forme solide ou liquide en fonction de la nature même des corps gras qui les composent. Ainsi, si au moins un corps gras constituant du SMEDDS® présente un point de fusion supérieur à la température ambiante, environ 25°C, alors le SMEDDS® sera solide à température ambiante. Au contraire, si au moins un corps gras constituant du SMEDDS® présente un point de fusion inférieur à environ 25°C, alors le SMEDDS® est liquide à température ambiante. En conséquence, les SMEDDS® peuvent être incorporés dans des gélules à l'état liquide, éventuellement à chaud puis, en fonction de la nature de leurs constituants, restent liquides ou deviennent semi-solides à température ambiante. Le procédé de fabrication est relativement simple puisqu'il consiste à mélanger l'ensemble des constituants, y compris le principe actif, à chaud ou à froid en fonction des caractéristiques physico-chimiques des corps gras.

Dans la suite de la description et dans les revendications :
. par l'expression "phase aqueuse", on désigne :
   - soit le milieu physiologique in vivo tel qu'il se présente après ingestion de la composition et dont le pH varie en fonction de l'état du tractus gastro-intestinal,
   - soit un milieu physiologique in vitro reconstitué, la micro-émulsion se formant alors au simple contact de la phase aqueuse, sans ingestion.
. l'ensemble des pourcentages est donné en mg/g.

Selon une première caractéristique de la composition de l'invention, la phase lipophile comprend un mélange de mono, di et triesters de glycérol et de mono et diesters de PEG avec au moins un acide gras choisi dans le groupe comprenant les acides gras en C₈-C₁₈ saturés et insaturés.

En pratique, ce mélange est obtenu par réaction d'alcoolyse de polyéthylène glycol de poids moléculaire compris entre 300 et 1500 et d'une huile végétale hydrogénée constituée elle-même d'un mélange en proportions variables, en fonction de sa nature, de mono, di et triglycérides d'au moins un des acides gras ci-avant décrits. Ce même mélange peut être également obtenu par estérification de glycérol et de PEG de poids moléculaire compris entre 300 et 1500 avec au moins un des acides gras précédemment décrits, ou encore par mélange d'esters de glycérol et de condensats d'éthylène oxyde avec au moins un desdits acides gras.

En pratique, la phase lipophile présente une HLB inférieure à 20, de préférence comprise entre 9 et 15 et représente entre 1 et 99 % en poids de la composition. Dans un premier mode de réalisation, la phase lipophile comprend majoritairement un mélange de mono, di et triesters de glycérol et de mono et diesters de PEG avec l'ensemble des acides gras saturés en C₈-C₁₈, a une HLB égale à 14 et représente entre 50 et 95 % en poids de la composition. En pratique, un tel mélange est obtenu par réaction d'alcoolyse de PEG de poids moléculaire compris entre 300 et 1500 avec une huile contenant des triglycérides lauriques en majorité. Un produit répondant à cette définition est le Gélucire® 44/14 commercialisé par le Demandeur. Ce produit est parfaitement défini dans la 3^{ème} édition de la pharmacopée européenne sous la définition macrogolglycérides lauriques. Dans un second mode de réalisation, la phase lipophile comprend un mélange de mono, di et triesters de glycol et de mono et diesters de PEG avec des acides gras en C₁₆-C₁₈ saturés et insaturés. Des produits répondant à cette définition sont les LABRAFIL M1944CS et LABRAFIL M2125CS commercialisés par le Demandeur et conformes aux monographies de la 3^{ème} édition de la pharmacopée européenne sous les dénominations respectivement "Oleoyl Macrogolglycerides" et "Linoleoyl Macrogolglycerides".

Par ailleurs et comme déjà dit, la phase tensioactive comprend un mélange de mono, di et triesters de glycérol et de mono, di et triesters de PEG avec les acides caprylique et caprique.

La phase tensioactive peut être obtenue de la même manière que précédemment par réaction d'alcoolyse à partir de polyéthylène glycol de poids moléculaire compris entre 200 et 600 et d'une fraction d'huile végétale hydrogénée riche en ester de glycérol avec les acides caprylique et caprique. La phase tensioactive peut également être obtenue par estérification de glycérol et de polyéthylène glycol avec les acides caprylique et caprique mais également par mélange d'ester de glycérol et de condensats d'éthylène oxyde avec les acides caprylique et caprique. En pratique, la phase tensioactive présente une HLB comprise entre 5 et 20.

Un produit correspondant à la définition de la phase tensioactive est le produit commercialisé par le demandeur sous la marque LABRASOL®, lequel correspond à la monographie de la 3^{ème} édition de la pharmacopée européenne intitulée "magrogol glycéride caprylocaprique". Dans un mode de réalisation avantageux, la phase tensioactive représente entre 1 et 30 % en poids de la composition.

Par ailleurs, et comme déjà dit, la phase co-tensioactive comprend au moins un ester d'alcool avec au moins un acide gras.

Sont plus particulièrement préférés les monoesters de propylène glycol choisis dans le groupe comprenant le monocaprilate de propylène glycol et le monolaurate de propylène glycol. Les produits commercialisés par le Demandeur et contenant des monoesters de propylène glycol et d'acide caprylique sont le CAPRYOL® 90 et le CAPRYOL® PGMC. De même, un produit commercialisé par le Demandeur et contenant du monolaurate de propylène glycol est le LAUROGLYCOL 90®.

Dans un premier mode de réalisation, la phase cotensioactive contient du monocaprylate de propylène glycol et représente entre 3 et 32 % en poids de la composition.

Dans un second mode de réalisation, la phase cotensioactive contient du monolaurate de propylène glycol et représente entre 1 et 8 % en poids de la composition. Dans ce cas de figure, on utilise avantageusement le LAUROGLYCOL® 90.

Comme déjà dit, le système auto-micro-émulsionable tel que précédemment décrit permet de diminuer l'effet de premier passage intestinal d'un certain nombre de principes actifs tels que par exemple ceux appartenant à la famille des statines, notamment la simvastatine. Dès lors et dans un mode de réalisation particulier, la statine est la simvastatine. Par ailleurs, pour être thérapeutiquement efficace, la simvastatine représente entre 0,1 et 6 % en poids de la composition, avantageusement 4 % en poids.

Dans un mode de réalisation particulier de l'invention, la composition pharmaceutique comprend en poids (mg/g) :
- entre 0,1 et 6 % de simvastatine,
- entre 52 et 70 % de GELUCIRE® 44/14,
- entre 5 et 30 % de LABRASOL®,
- entre 15 et 30 % de monocaprylate de propylène glycol.

Dans une première forme de réalisation, le monocaprylate de propylène glycol contenu dans cette composition est constitué de CAPRYOL® PGMC représentant entre 15 et 25 % en poids de la composition. Dans une seconde forme de réalisation, le monocaprylate est constitué de CAPRYOL® 90 et représente entre 20 et 30 % en poids de la composition.

Dans une forme préférée, la composition comprend :
- 4% de simvastatine,
- 65,2 % de GELUCIRE® 44/14,
- 10,3 % de LABRASOL®,
- 20,5 % de CAPRYOL PGMC.

Alternativement, la composition comprend :
- 4% de simvastatine,
- 57,6 % de GELUCIRE® 44/14,
- 12,8 % de LABRASOL®,
- 25,6 % de CAPRYOL® 90.

Selon un autre mode de réalisation, la composition pharmaceutique comprend en poids (en mg/g) :
- 0,1 à 6 % de simvastatine,
- entre 52 et 70 % de GELUCIRE® 44/14,
- entre 6 et 30 % de LABRASOL®,
- entre 1 et 8 % de LAUROGLYCOL® 90.

Avantageusement, la composition de l'invention contient :
- 4 % de simvastatine,
- 65,3 % de GELUCIRE® 44/14,
- 24,6 % de LABRASOL®,
- 6,1 % de LAUROGLYCOL® 90.

En effet, le Demandeur a constaté que l'utilisation d'esters capryliques en tant que phase co-tensioactive dans les compositions du type SMEDDS®, non décrite dans le document EP-A-677 115, permettait d'obtenir une zone de microémulsion plus longue, c'est-à-dire d'obtenir une microémulsion en présence de fortes proportions d'eau par rapport à l'utilisation par exemple d'esters lauriques du propylène glycol. Le système SMEDDS® permet donc, en présence d'ester capryliques du propylène glycol en tant que phase co-tensioactive, d'améliorer la solubilité des principes actifs hydrophobes et d'obtenir une formule stable en présence de fortes proportions d'eau, compatible avec le volume de liquide physiologique de l'organisme.

Les phases lipophile, tensioactive et co-tensioactive correspondent aux définitions et sont utilisées dans les mêmes proportions que précédemment décrites en limitant toutefois l'utilisation des esters de propylène glycol aux esters capryliques du propylène glycol tels que ceux commercialisés par exemple par le Demandeur sous la dénomination CAPRYOL® PGMC et CAPRYOL® 90.

Dans un mode de réalisation avantageux, le ratio TA/CoTA est égal à 0,5.

L'invention et les avantages qui en découlent ressortiront mieux de l'exemple de réalisation suivant à l'appui des figures annexées.
La figure 1 représente la concentration plasmatique moyenne de simvastatine en fonction du temps.
La figure 2 représente la concentration plasmatique moyenne d'hydroxysimvastatine en fonction du temps.
La figure 3 est un diagramme ternaire d'une formule SMEDDS® comprenant du laurate de propylène glycol, en tant que CoTA.
Les figures 4' 5 et 6 sont des diagrammes ternaires d'une formule SMEDDS® comprenant un caprylate de propylène glycol, ou un ester oléique du polyglycérol en tant que CoTA.

### Exemple 1

On fabrique les trois formulations suivantes :

| ***COMPOSANTS*** | ***FORMULE 1*** | ***FORMULE 2*** | ***FORMULE 3*** |
|---|---|---|---|
| ***SIMVASTATINE*** | 4.0 % | 4.0 % | 4.0 % |
| ***LABRASOL*** | 10.3 % | 12.8 % | 24.6 % |
| ***GÉLUCIRE® 44*/*14*** | 65.2 % | 57.6 % | 65.3 % |
| ***CAPRYOL® PGMC*** | 20.5 % | - | - |
| ***CAPRYOL® 90*** | - | 25.6 % | - |
| ***LAUROGLYCOL® 90*** | - | - | 6.1 % |
| **TOTAL** | 100 % | 100 % | 100 % |

Chacun des constituants des formules sont mélangés à température ambiante sous agitation comprise entre 60 et 100 tour/min.

### Exemple 2 : Diminution de l'effet de barrière intestinale avec le produit objet de l'invention par rapport à la simvastatine seule à partir de microsomes intestinaux humains.

Le test est effectué in vitro à partir de microsomes intestinaux humains contenant :
- 20 mg/ml de protéines,
- 0,09 nmol/mg de cytochromes P450,
- 0,77 nmol/min/mg de cytochromes P450 3 A 4.

1 mg/ml de microsome est ensuite mis en présence :
. d'un système régénérant présentant la composition suivante :
   - NADPH : 1 mmol
   - Glucose 6-phosphate dehydrogenase : 2 unités/ml
   - Glucose 6-phosphate : 10 mmol
. Phosphate de potassium, pH 7,4 : 100 mmol
. Chlorure de magnésium : 10 mmol puis équilibré à 37° C pendant 3 minutes.

On initie ensuite la réaction en ajoutant 12 µmol de simvastatine ou de la composition de l'invention au mélange réactionnel. Des aliquotes de 100 µl sont mélangés avec une solution de 400 µl d'un mélange 50/50 de glace et d'acétonitrile à 0, 15, 30, 60, 120 et 180 minutes. On teste parallèlement la testostérone en tant que composant de contrôle.

Le taux de simvastatine restant est ensuite quantifié par HPLC et spectrométrie de masse.
Les conditions de la mise en oeuvre de la HPLC sont les suivantes :
- colonne : Hypersil BDS C18, 30x2 mm i.d., 3µm,
- tampon : 25 mmol d'hydroxyde d'ammonium ajusté à un pH de 3,5 avec de l'acide formique,
- phase mobile : A - 10 % de tampon et 90 % d'eau, B -10 % de tampon et 90 % d'acétonitrile,
- gradient : 0 % de B à 100 % de B en 3 minutes, rééquilibration pendant 2 minutes,
- débit : 300 µl/min
- volume d'injection : 10 µl

Le spectromètre de masse utilisé est connu sous la référence PE SCIEX 150.

Les résultats figurent dans le tableau suivant. Au bout de 15 minutes, la testostérone a complètement disparu du milieu cellulaire. En revanche, pour les trois compositions de l'invention, entre 20 et 23 % de simvastatine reste dans la circulation malgré l'effet de premier passage intestinal.

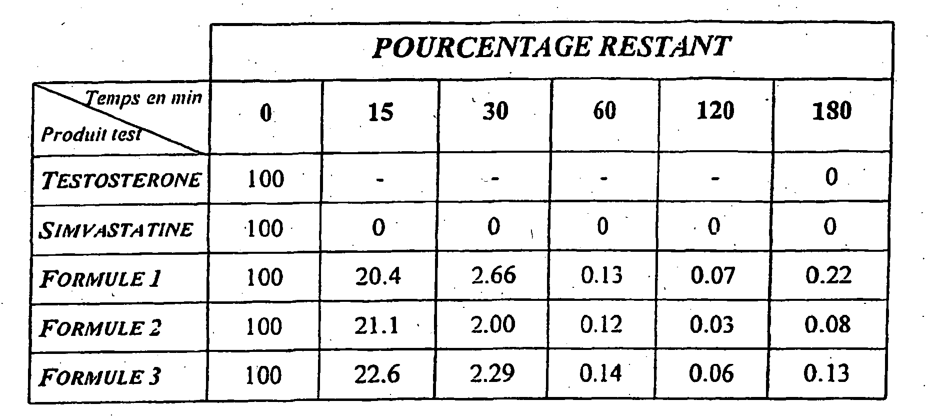

On en déduit donc que le SMEDDS® permet d'augmenter la vitesse de solubilisation de la simvastatine, donc de saturer plus rapidement les sites enzymatiques et donc de rendre la simvastatine en excès directement disponible par augmentation de la vitesse d'absorption.

### Exemple 3 : Comparaison des biodisponibilités relatives in vivo entre les formules 1, 2, 3 et une formule de référence, à savoir le ZOCOR®.

Pour évaluer l'influence de différentes concentrations de la composition de l'invention sur la biodisponibilité relative orale de la simvastatine, quatre chiens mâles Beagle ont été traités selon un modèle croisé (sur 4 périodes, une formulation par période et par chien, une semaine d'intervalle entre chaque période) avec les trois formulations 1, 2, 3 et une formulation de référence, le ZOCOR® commercialisé par les Laboratoires MERCK, SHARP & DOHME-CHIBRET.

Les formulations ont été administrées sous forme de capsule, chacune comprenant 40 mg de simvastatine. Chaque chien a reçu deux capsules correspondant donc à une dose totale de 80 mg. La dose administrée de 80 mg par chien est supposée donner des profils de concentration plasmatique en simvastatine comparable à ceux observés chez l'humain à des doses thérapeutiques élevés (80 mg).

Chaque chien a subi un prélèvement sanguin avant le traitement et à des temps successifs de 15 minutes, 30 minutes, 1 heure, 1 heure et demi, 2 heures, 3 heures, 4 heures, 6 heures, 8 heures, 12 heures et 24 heures. Les concentrations plasmatiques en simvastatine et en hydroxysimvastatine ont été déterminées par méthode analytique HPLC/MS.

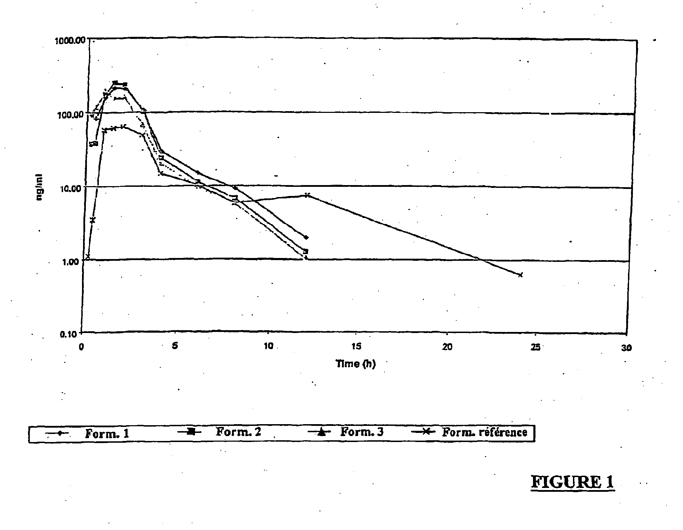

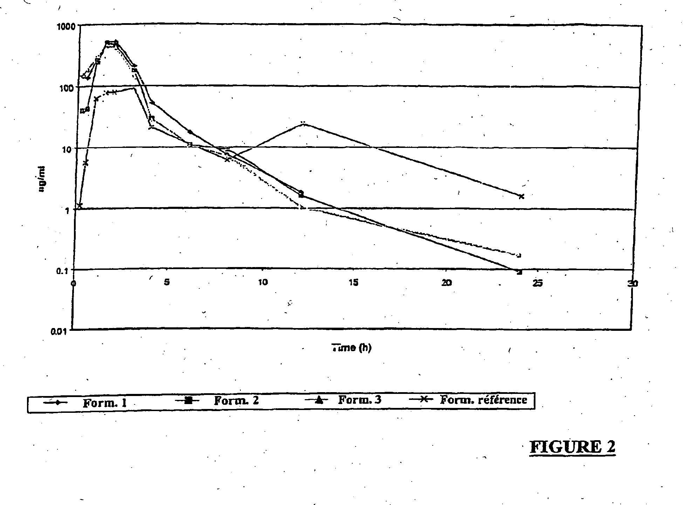

Les résultats apparaissent sur les figures 1 et 2, lesquelles représentent les concentrations plasmatiques moyennes en simvastatine (figure 1) et en hydroxysimvastatine (figure 2) en fonction du temps pour les trois formulations et la formule de référence (ZOCOR®).

Après administration du ZOCOR®, on observe un retard dans l'absorption de la simvastatine. En effet, la simvastatine et l'hydroxysimvastatine sont retrouvées dans le plasma chez tous les animaux à partir seulement du troisième échantillon, c'est-à-dire 1 heure après l'administration. Au contraire, les taux plasmatiques de simvastatine et d'hydroxysimvastatine sont détectées dès la 15^{ème} minute après administration des formules 1, 2, 3. En d'autres termes, la composition de l'invention augmente le taux d'absorption.

Dans le tableau ci-après, figurent les paramètres moyens cinétiques (Cmax, Tmax) et d'absorption (Vmax, T50%, T90%) pour la formule de référence ZOCOR® et les formules 1 à 3. Dans ce tableau, les paramètres ont les significations suivantes :
Cmax : taux plasmatique maximum (ng/ml)
Tmax : durée pour obtenir Cmax (h)
Vmax : taux maximum d'absorption (% dose/h)
T50 % : durée pour absorber 50 % de la dose
T90 % : durée pour absorber 90 % de la dose

| **Paramètres** | **Reference** | **Formule 1** | **Formule 2** | **Formule 3** |
|---|---|---|---|---|
| **Cmax** | 63.945 | 215.2125 | 249.49 | 203.84 |
| **Tmax** | 1 | 1.5 | 1 | 0.5 |
| **Vmax (%h)** | 19.71 | 57.84 | 62.14 | 62.91 |
| **T50% (h)** | 3 | 1.5 | 1.5 | 1 |
| **T90% (h)** | 22 | 5 | 3.5 | 3.5 |

Comme le montre le tableau ci-dessus, le taux d'absorption et trois fois plus élevé pour les formules de l'invention par rapport au ZOCOR®. En conséquence, on retrouve une concentration plasmatique maximum pour les formules de l'invention bien supérieure à celle de ZOCOR®.

La différence la plus significative entre les formulations 1, 2, 3 et le ZOCOR® concerne l'amélioration de l'absorption. En effet, après administration de la formulation 1, l'aire sous la courbe moyenne (AUC de la simvastatine et de l'hydroxysimvastatine) est supérieure de deux à trois aux valeurs correspondantes du ZOCOR®.

Dans le tableau ci-après, figurent les taux d'absorption en fonction du temps de la simvastatine.

| | **Taux moyen d'absorption (V)** *(% de la dose* / *h)* | | | |
|---|---|---|---|---|
| **T(h)** | **Ref.** | **1** | **2** | **3** |
| 0 | 0.00 | 0.00 | 0.00 | 0.00 |
| 0.25 | 0.67 | 57.54 | 23.98 | 62.91 |
| 0.5 | 1.72 | 18.06 | 10.65 | 26.67 |
| 1 | 19.71 | 47.56 | 57.13 | 53.14 |
| 1.5 | 12.52 | 48.33 | 62.14 | 26.36 |
| 2 | 13.29 | 40.55 | 45.08 | 33.09 |
| 3 | 8.38 | 18.66 | 16.94 | 11.32 |
| 4 | 2.03 | 3.01 | 2.82 | 2.56 |
| 6 | 2.21 | 3.61 | 2.93 | 2.29 |
| 8 | 1.33 | 2.27 | 1.60 | 1.28 |
| 12 | 1.38 | 0.85 | 0.52 | |
| 24 | 1.01 | | | |

Comme le montre ce tableau, la vitesse d'absorption de la simvastatine est près de 100 fois supérieure pour les formules 1 et 3 par rapport à la formule de référence. La formulation 3 montre quant à elle qu'on peut faire varier la nature des constituants et ainsi agir directement sur le taux d'absorption. Ces résultats démontrent donc que l'on agit sur la vitesse de solubilisation de l'actif.

Par ailleurs, l'index de bioéquivalence relative résultant de la somme de l'aire sous la courbe (AUC de la simvastatine et l'hydroxysimvastatine pour la formulation 1 Vs le ZOCOR®) est de 3,26. La bioéquivalence relative correspondant à la formule 2 est de 2,88, et la formule 3 est de 2,66.

Dès lors, même si une faible diminution de la biodisponibilité relative entre les formules 1, 2 et 3 est observée, les différentes concentrations des composants constituant ces formules n'induisent pas de variation de la biodisponibilité relative de la simvastatine chez le chien.

### Exemple 4

Cet exemple vise à démontrer la capacité des SMEDDS® à former une microémulsion en présence d'une forte proportion d'eau lorsqu'un ester caprylique du propylène glycol est utilisé en tant que phase co-tensioactive (comparé aux esters lauriques (formules 1, 2, 3) ou aux esters oléiques du polyglycérol.

Les trois formules suivantes sont testées.

| **FORMULES** | **1** | **2** | **3** | **4** |
|---|---|---|---|---|
| **TA** | LABRASOL | LABRASOL | LABRASOL | LABRASOL |
| **Phase lipophile** | GELUCIRE 44/14 | GELUCIRE 44/14 | GELUCIRE 44/14 | GELUCIRE 44/14 |
| **CoTA** | LAUROGLYCOL 90 | CAPRYOL90 | CAPRYOL PGMC | PLUROL OLEIQUE |
| **Ratio TA/CoTA** | 0,5 | 0,5 | 0,5 | 0,5 |

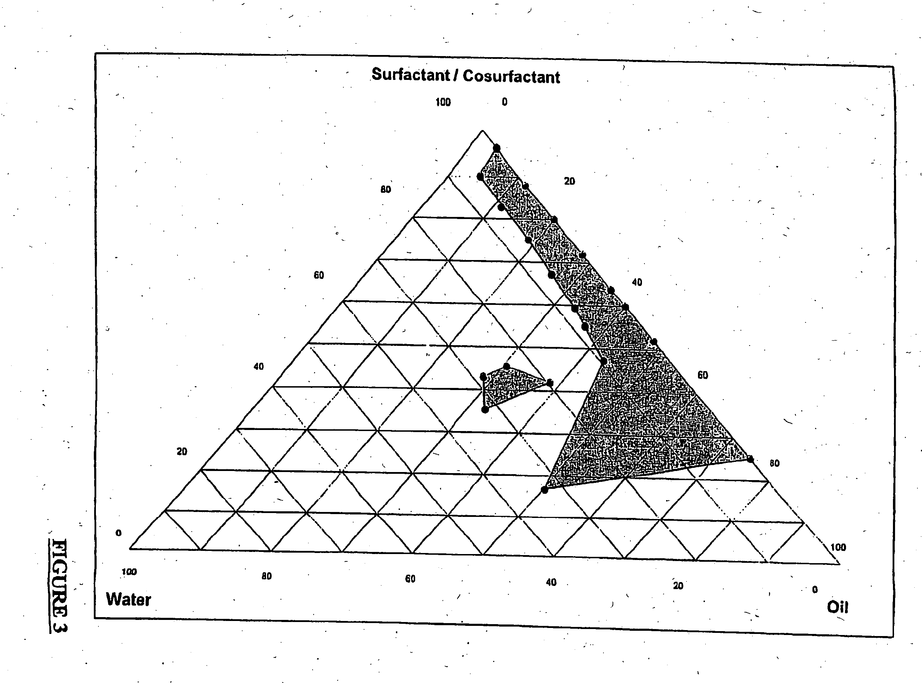

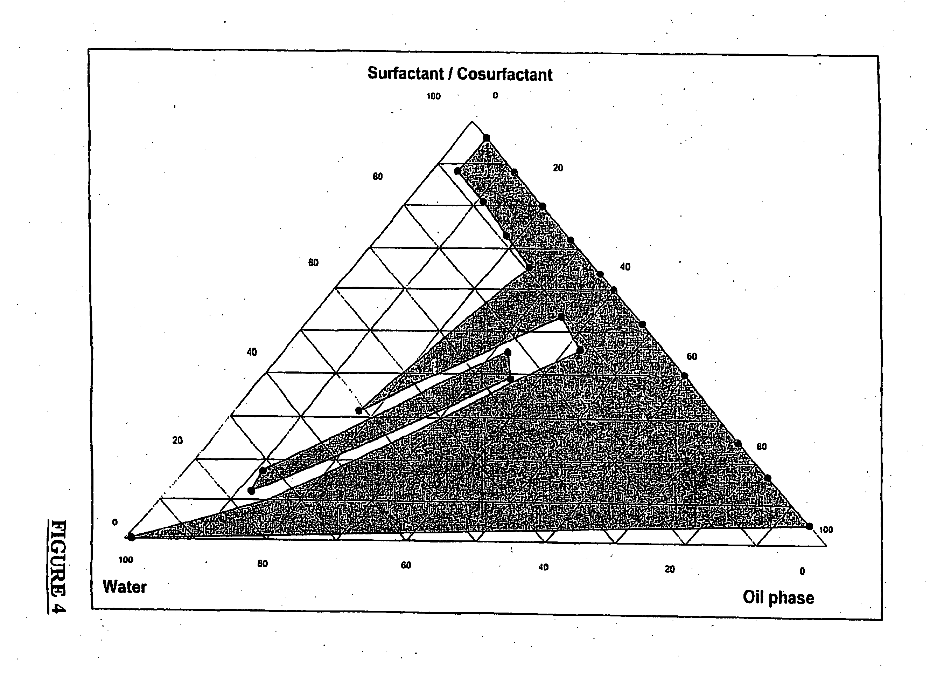

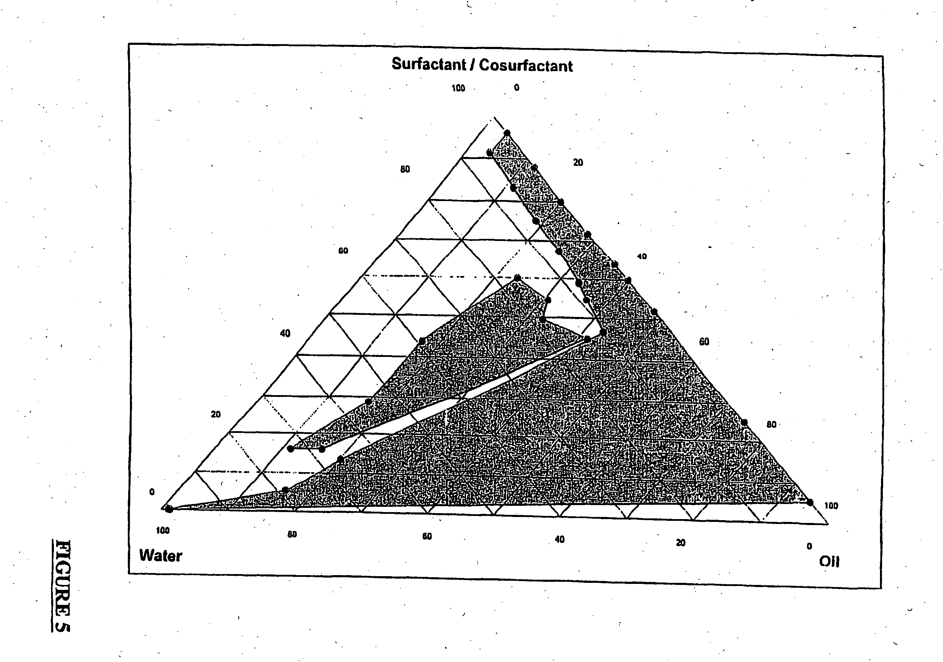

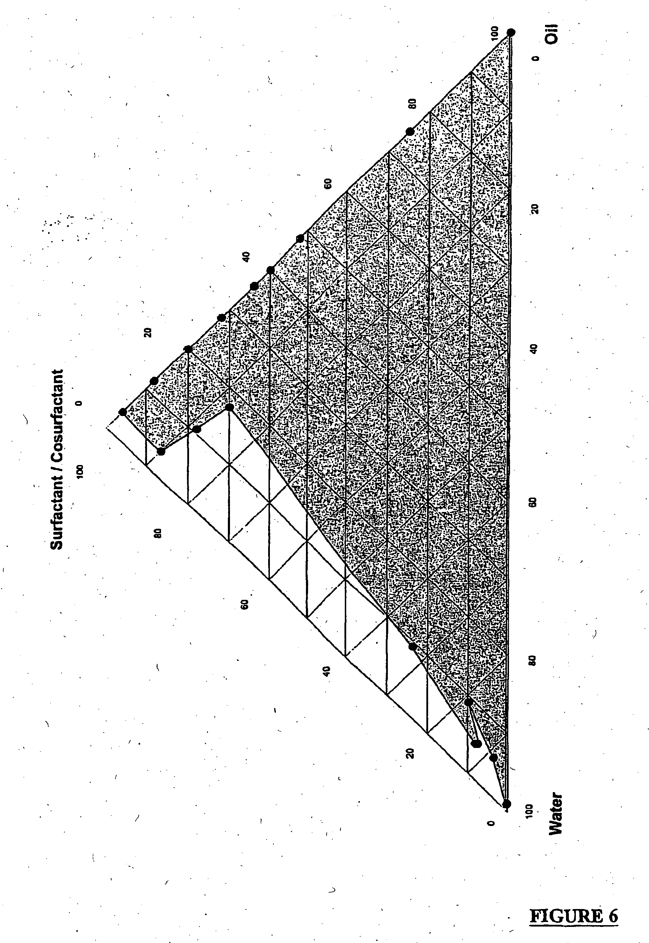

Sur les figures 3, 4, 5 et 6 sont représentés les diagrammes ternaires correspondant aux formules 1 à 4.

Les zones hachurées correspondent aux zones et micro-émulsions. Comme le montrent ces figures, la zone de micro-émulsion est plus large lorsqu'on utilise un ester caprylique en lieu et place d'un ester laurique de propylène glycol ou d'un ester oléique du polyglycerol.

## Revendications

1. Utilisation d'un système auto-micro-émulsionable au contact d'une phase aqueuse, comprenant :
- une quantité thérapeutiquement efficace d'un principe actif susceptible de subir un effet de premier passage intestinal;
- une phase lipophile comprenant un mélange de mono, di, et triesters de glycérol et de mono et diesters de PEG avec au moins un acide gras choisi dans le groupe comprenant les acides gras en C₈-C₁₈ ;
- une phase tensioactive (TA) comprenant un mélange de mono, di et triesters de glycérol et de mono et diesters de PEG avec les acides caprylique (C₈) et caprique (C₁₀) ;
- une phase cotensioactive (CoTA) comprenant au moins un ester d'alcool avec au moins un acide gras choisi dans le groupe comprenant les esters caprylique du propylène glycol, les esters laurique du propylène glycol et les esters oléïque du polyglycérol,
- le ratio TA/CoTA étant compris entre 0,2 et 6,
pour la préparation d'une composition pharmaceutique à usage oral destinée à diminuer le métabolisme intestinal du principe actif.

2. Utilisation selon la revendication 1, **caractérisée en ce que** la phase lipophile comprend un mélange de mono, di et triesters de glycérol et de mono et diesters de PEG avec l'ensemble des acides gras saturés en C₈-C₁₈, ledit mélange présentant une HLB égale à 14 et représentant entre 50 et 95 % en poids de la composition.

3. Utilisation selon la revendication 1, **caractérisée en ce que** la phase tensioactive représente entre 1 et 30 % en poids du mélange.

4. Utilisation selon la revendication 1, **caractérisée en ce que** la phase cotensioactive est un monoester de propylène glycol choisi dans le groupe comprenant le monocaprylate de propylène glycol et le monolàurate de propylène glycol.

5. Utilisation selon la revendication 4, **caractérisée en ce que** lorsque las phase tensioactive contient du monocaprylate de propylène glycol, elle représente entre 3 et 32 % en poids de la composition.

6. Utilisation selon la revendication 4, **caractérisée en ce que** lorsque la phase cotensioactive contient du monolaurate de propylène glycol, elle représente entre 1 et 8 % en poids de la composition.

7. Utilisation selon la revendication 1, **caractérisés en ce que** le principe actif appartient à la famille des statines.

8. Utilisation selon la revendication 7, **caractérisée en ce que** la statine est la simvastatine.

9. Utilisation selon la revendication 8, **caractérisée en ce que** la simvastatine représente entre 0,1 et 6 % en poids de la composition, avantageusement 4 % en poids.

10. Utilisation selon la revendication 1, **caractérisée en ce que** le système auto-micro-émulsionable comprend en poids :
- entre 0,1 et 6 % de simvastatine,
- entre 52 et 70 % de GELUCIRE^{®} 44/14 (macrogolglycérides, lauriques),
- entre 5 et 30 % de LABRASOL^{®} (macrogolglycéride caprylodaprique),
- entre 15 et 30 % de monocaprylate de propylène glycol.

11. Utilisation selon la revendication 10, **caractérisée en ce que** le monocaprylate de propylène glycol est constitué de CAPRYOL^{®} PGMC représentant entre 15 et 25 % en poids de la composition.

12. Utilisation selon la revendication 10, **caractérisée en ce que** le monocaprylate de propylène glycol est constitué de CAPRYOL^{®} 90 représentant entre 20 et 30 % en poids de la composition.

13. Utilisation selon la revendication 1, **caractérisée en ce que** le système auto-micro-émulsionable comprend en poids :
- entre 0,1 à 6 % de simvastatine,
- entre 52 et 70 % de GELUCIRE^{®} 44/14 (macrogolglycérides lauriques),
- entre 5 et 30 % de LABRASOL^{®} (macrogolglycéride caprylocaprique),
- entre 1 et 8 % de LAUROGLYCOL^{®} 90 (monolaurate de propylène glycol).

14. Composition pharmaceutique à usage oral se présentant sous forme d'un système auto-micro-émulsionnable au contact d'une phase aqueuse comprenant :
- une quantité efficace d'un principe actif susceptible de subir un effet de premier passage intestinal,
- une phase lipophile comprenant un mélange de mono, di et tri esters de glycérol et de mono et di esters de PEG avec au moins un acide gras choisi dans le groupe comprenant les acides gras en C₈-C₁₈,
- une phase tensioactive (TA) comprenant un mélange de mono, di et tri esters de glycérol et de mono et di esters de PEG avec les acides caprylique C₈ et caprique C₁₀,
- une phase co-tensioactive (CoTA) comprenant au moins un ester d'alcool avec au moins un acide gras,
- le ratio TA/CoTA étant compris entre 0,2 et 6,
- à l'exclusion de la formule constituée de 1,4 % d'isotrétinoïne, de 9,7 % de Labrasol®, de 13,9 % de Capryol®, de 75 % de Gelucire® 44/14,
ainsi que la formule constituée de 5% de progestérone, 8,5% de Labrasol^{®}, de 65% de Gelucire^{®} 44/14, de 17,5% de Capryol^{®} PGMC, de 2% de Labrafil^{®} M 1944, et de 2% de Labrafac^{®} C, **caractérisée en ce que** l'ester d'alcool avec au moins un acide gras constitutif de la phase co-tensioactive est choisi dans le groupe comprenant les esters capryliques du propylène glycol.

15. Composition selon la revendication 14, **caractérisée en ce que** la phase lipophile comprend un mélange de mono, di et tri esters de glycérol et de mono et di esters de PEG avec l'ensemble des acides gras saturés en C₈-C₁₈, ledit mélange présentant une HLB égale à 14 et représentant entre 50 et 95 % en poids de la composition.

16. Composition selon la revendication 14, **caractérisée en ce que** la phase tensioactive représente entre 1 et 30 % en poids du mélange.

17. Composition selon la revendication 14, **caractérisée en ce que** la phase co-tensioactive représente entre 3 et 32 % en poids de la composition.

18. Composition selon la revendication 14, **caractérisée en ce que** le principe actif appartient à la famille des statines.

19. Composition selon la revendication 18, **caractérisée en ce que** la statine est la simvastatine.

20. Composition selon la revendication 19, **caractérisée en ce que** la simvastatine représente entre 0,1 et 6 % en poids de la composition, avantageusement 4 % en poids.

21. Composition selon la revendication 14, **caractérisée en ce qu'**elle comprend en poids :
- entre 0,1 et 6 % de simvastatine,
- entre 52 et 70 % de GELUCIRE 44/14 (macrogolglycéride laurique),
- entre 5 et 30 % de LABRASOL (macrogolglycéride caprylocaprique),
- entre 15 et 30 % de monocaprylate de propylène glycol.

22. Composition selon la revendication 21, **caractérisée en ce que** le monocaprylate de propylène glycol est constitué de CAPRYOL^{®} PGMC représentant entre 15 et 25 % en poids de la composition.

23. Composition selon la revendication 21, **caractérisée, en ce que** le monocaprylate de propylène glycol est constitué de CAPRYOL^{®} 90 et représente entre 20 et 30 % en poids de la composition.

24. Composition selon la revendication 14, **caractérisée en ce que** le ratio TA/CoTA est. égal à 0,5.

## Claims

1. Use of a system which is self-microemulsifying on contact with an aqueous phase, comprising:
- a therapeutically effective amount of an active principle liable to undergo a first intestinal passage effect;
- a lipophilic phase comprising a mixture of glycerol mono-, di- and triesters and of PEG mono- and diesters with at least one fatty acid chosen from the group comprising C₈-C₁₈ fatty acids;
- a surfactant phase (TA) comprising a mixture of glycerol mono-, di- and triesters and of PEG mono- and diesters with caprylic acid (C₈) and capric acid (C₁₀);
- a co-surfactant phase (CoTA) comprising at least one ester of alcohol with at least one fatty acid chosen from the group comprising caprylic esters of propylene glycol, lauric esters of propylene glycol and oleic esters of polyglycerol,
- the ratio TA/CoTA being between 0.2 and 6,
for the preparation of an oral pharmaceutical composition able to reduce the intestinal metabolism of the active principle.

2. Use according to Claim 1, **characterized in that** the lipophilic phase comprises a mixture of glycerol mono-, di- and triesters and of PEG mono- and diesters with the combination of saturated C₈-C₁₈ fatty acids, the said mixture having an HLB value equal to 14 and representing between 50 and 95% by weight of the composition.

3. Use according to claim 1, **characterized in that** the surfactant phase represents between 1% and 30% by weight of the mixture.

4. Use according to claim 1, **characterized in that** the co-surfactant phase is a monoester of propylene glycol chosen from the group comprising propylene glycol monocaprylate and propylene glycol monolaurate.

5. Use according to Claim 4, **characterized in that**, when the surfactant phase contains propylene glycol monocaprylate, it represents between 3% and 32% by weight of the composition.

6. Use according to Claim 4, **characterized in that**, when the co-surfactant phase contains propylene glycol monolaurate, it represents between 1% and 8% by weight of the composition.

7. Use according to claim 1, **characterized in that** the active principle belongs to the statin family.

8. Use according to Claim 7, **characterized in that** the statin is simvastatin.

9. Use according to Claim 8, **characterized in that** the simvastatin represents between 0.1% and 6% by weight of the composition and advantageously 4% by weight.

10. Use according to claim 1, **characterized in that** the self-microemulsifying system comprises by weight:
- between 0.1% and 6% of simvastatin,
- between 52% and 70% of Gélucire® 44/14 (lauric macrogolglycerides),
- between 5% and 30% of Labrasol® (caprylocapric macrogolglycerides),
- between 15% and 30% of propylene glycol monocaprylate.

11. Use according to Claim 10, **characterized in that** the propylene glycol monocaprylate consists of Capryol® PGMC representing between 15% and 25% by weight of the composition.

12. Use according to Claim 10, **characterized in that** the propylene glycol monocaprylate consists of Capryol® 90 representing between 20% and 30% by weight of the composition.

13. Use according to claim 1, **characterized in that** the self-microemulsifying system comprises by weight:
- between 0.1% and 6% of simvastatin,
- between 52% and 70% of Gélucire® 44/14 (lauric macrogolglycerides),
- between 5% and 30% of Labrasol® (caprylocapric macrogolglycerides),
- between 1% and 8% of Lauroglycol® 90 (propylene glycol monolaurate).

14. Pharmaceutical composition for oral use that is in the form of a system which is self-microemulsifying on contact with an aqueous phase, comprising:
- a therapeutically effective amount of an active principle liable to undergo a first intestinal passage effect;
- a lipophilic phase comprising a mixture of glycerol mono-, di- and triesters and of PEG mono- and diesters with at least one fatty acid chosen from the group comprising C₈-C₁₈ fatty acids;
- a surfactant phase (TA) comprising a mixture of glycerol mono-, di- and triesters and of PEG mono- and diesters with caprylic acid (C₈) and capric acid (C₁₀);
- a co-surfactant phase (CoTA) comprising at least one ester of alcohol with at least one fatty acid;
- the ratio TA/CoTA being between 0.2 and 6,
- except the formula consisting of 1,4% of isotretinoin, 9,7% of Labrasol^{®}, 13,9% of Capryol^{®}, 75% of Gélucire^{®} 44/14, and the formula consisting of 5% of progesterone, 8,5% of Labrasol^{®}, 65% of Gélucire^{®}, 17,5% of Capryol^{®} PGMC, 2% of Labrafil^{®} M1944, and 2% of Labrafac^{®} CC,
**characterized in that** the ester of alcohol with at least one fatty acid present in the co-surfactant phase is chosen from the group comprising caprylic esters of propylene glycol.

15. Composition according to Claim 14, **characterized in that** the lipophilic phase comprises a mixture of glycerol mono-, di- and triesters and of PEG mono- and diesters with the combination of saturated C₈-C₁₈ fatty acids, the said mixture having an HLB value equal to 14 and representing between 50 and 95% by weight of the composition.

16. Composition according to claim 14, **characterized in that** the surfactant phase represents between 1% and 30% by weight of the mixture.

17. Composition according to claim 14, **characterized in that** the co-surfactant phase represents between 3% and 32% by weight of the mixture.

18. Composition according to claim 14, **characterized in that** the active principle belongs to the statin family.

19. Composition according to Claim 18, **characterized in that** the statin is simvastatin.

20. Composition according to Claim 19, **characterized in that** the simvastatin represents between 0.1% and 6% by weight of the composition and advantageously 4% by weight.

21. Composition according to claim 14, **characterized in that** it comprises by weight:
- between 0.1% and 6% of simvastatin,
- between 52% and 70% of Gélucire® 44/14 (lauric macrogolglycerides),
- between 5% and 30% of Labrasol® (caprylocapric macrogolglycerides),
- between 15% and 30% of propylene glycol monocaprylate (propylene glycol monolaurate).

22. Composition according to Claim 21, **characterized in that** the propylene glycol monocaprylate consists of Capryol® PGMC representing between 15% and 25% by weight of the composition.

23. Composition according to Claim 21, **characterized in that** the propylene glycol monocaprylate consists of Capryol® 90 representing between 20% and 30% by weight of the composition.

24. Composition according to Claim 14, **characterized in that** the ratio TA/CoTA is equal to 0,5.

## Patentansprüche

1. Verwendung eines Systems, welches bei Kontakt mit einer wäßrigen Phase selbsttätig eine Mikroemulsion bilden kann und welches folgendes umfaßt:
- eine therapeutisch wirksame Menge eines Wirkstoffs, der bei seiner ersten Darmpassage Wirkungen ausgesetzt werden kann;
- eine lipophile Phase, welche ein Gemisch aus Glyzerinmono-, -di- und -triestern und PEG-Mono- und -Diestern mit mindestens einer aus der die Fettsäuren aus C₈-C₁₈ umfassenden Gruppe ausgewählten Fettsäure umfaßt;
- eine Tensid-Phase (TA), welche ein Gemisch aus Glyzerinmono-, - di- und -triestern und PEG-Mono- und -Diestern mit Caprylsäure (C₈) und Caprinsäure (C₁₀) umfaßt;
- eine Cotensid-Phase (CoTA), welche mindestens einen Alkoholester mit mindestens einer aus der die Caprylester von Propylenglykol, die Laurylester von Propylenglykol und die Ölsäureester von Polyglyzerin umfassenden Gruppe ausgewählten Fettsäure umfaßt;
- wobei das Verhältnis TA/CoTA zwischen 0,2 und 6 liegt,
für die Zubereitung einer oral anzuwendenden pharmazeutischen Zusammensetzung, welche dazu bestimmt ist, den intestinalen Metabolismus des Wirkstoffs zu verringern.

2. Verwendung nach Anspruch 1, **dadurch gekennzeichnet, daß** die lipophile Phase ein Gemisch aus Glyzerinmono-, -di- und -triestern und PEG-Mono- und -Diestern mit der Gesamtheit der gesättigten Fettsäuren aus C₈-C₁₈ umfaßt, wobei das genannte Gemisch einen HLB-Wert gleich 14 aufweist und zwischen 50 und 95 Gewichtsprozent der Zusammensetzung darstellt.

3. Verwendung nach Anspruch 1, **dadurch gekennzeichnet, daß** die Tensid-Phase zwischen 1 und 30 Gewichtsprozent des Gemisches darstellt.

4. Verwendung nach Anspruch 1, **dadurch gekennzeichnet, daß** die Cotensid-Phase ein aus der Propylenglykolmonocaprylat und Propylenglykolmonolaurat umfassenden Gruppe ausgewählter Propylenglykolmonoester ist.

5. Verwendung nach Anspruch 4, **dadurch gekennzeichnet, daß**, wenn die Tensid-Phase Propylenglykolmonocaprylat enthält, sie zwischen 3 und 32 Gewichtsprozent der Zusammensetzung darstellt.

6. Verwendung nach Anspruch 4, **dadurch gekennzeichnet, daß**, wenn die Cotensid-Phase Propylenglykolmonolaurat enthält, sie zwischen 1 und 8 Gewichtsprozent der Zusammensetzung darstellt.

7. Verwendung nach Anspruch 1, **dadurch gekennzeichnet, daß** der Wirkstoff der Familie der Statine angehört.

8. Verwendung nach Anspruch 7, **dadurch gekennzeichnet, daß** das Statin Simvastatin ist.

9. Verwendung nach Anspruch 8, **dadurch gekennzeichnet, daß** das Simvastatin zwischen 0,1 und 6 Gewichtsprozent, vorteilhafterweise 4 Gewichtsprozent, der Zusammensetzung darstellt.

10. Verwendung nach Anspruch 1, **dadurch gekennzeichnet, daß** das System, welches selbsttätig eine Mikroemulsion bilden kann, folgende Gewichtsanteile umfaßt:
- zwischen 0,1 und 6% Simvastatin,
- zwischen 52 und 70% GELUCIRE® 44/14 (Lauryl-Macrogolglyzeride),
- zwischen 5 und 30% LABRASOL® (Capryl-Caprin-Macrogolglyzerid),
- zwischen 15 und 30% Propylenglykolmonocaprylat.

11. Verwendung nach Anspruch 10, **dadurch gekennzeichnet, daß** das Propylenglykolmonocaprylat aus CAPRYOL® PGMC besteht, welches zwischen 15 und 25 Gewichtsprozent der Zusammensetzung darstellt.

12. Verwendung nach Anspruch 10, **dadurch gekennzeichnet, daß** das Propylenglykolmonocaprylat aus CAPRYOL® 90 besteht, welches zwischen 20 und 30 Gewichtsprozent der Zusammensetzung darstellt.

13. Verwendung nach Anspruch 1, **dadurch gekennzeichnet, daß** das System, welches selbsttätig eine Mikroemulsion bilden kann, folgende Gewichtsanteile umfaßt:
- zwischen 0,1 und 6% Simvastatin,
- zwischen 52 und 70% GELUCIRE® 44/14 (Lauryl-Macrogolglyzeride),
- zwischen 5 und 30% LABRASOL® (Capryl-Caprin-Macrogolglyzerid),
- zwischen 1 und 8% LAUROGLYCOL® 90 (Propylenglykolmonolaurat).

14. Oral anzuwendende pharmazeutische Zusammensetzung in der Form eines Systems, welches bei Kontakt mit einer wäßrigen Phase selbsttätig eine Mikroemulsion bilden kann und welches folgendes umfaßt:
- eine wirksame Menge eines Wirkstoffs, der bei seiner ersten Darmpassage Wirkungen ausgesetzt werden kann,
- eine lipophile Phase, welche ein Gemisch aus Glyzerinmono-, -di- und -triestern und PEG-Mono- und -Diestern mit mindestens einer aus der die Fettsäuren aus C₈-C₁₈ umfassenden Gruppe ausgewählten Fettsäure umfaßt,
- eine Tensid-Phase (TA), welche ein Gemisch aus Glyzerinmono-, - di- und -triestern und PEG-Mono- und -Diestern mit C₈-Caprylsäure und C₁₀-Caprinsäure umfaßt,
- eine Cotensid-Phase (CoTA), welche mindestens einen Alkoholester mit mindestens einer Fettsäure umfaßt,
- wobei das Verhältnis TA/CoTA zwischen 0,2 und 6 liegt,
- mit Ausschluß der Rezeptur, welche aus 1,4% Isotretinoin, 9,7% Labrasol®, 13,9% Capryol®, 75% Gelucire® 44/14 besteht, sowie der Rezeptur, welche aus 5% Progesteron, 8,5% Labrasol®, 65% Gelucire®, 17,5% Capryol® PGMC, 2% Labrafil® M 1944 und 2% Labrafac® CC besteht,
**dadurch gekennzeichnet, daß** der die Cotensid-Phase bildende Alkoholester mit mindestens einer Fettsäure aus der die Caprylester von Propylenglykol umfassenden Gruppe ausgewählt ist.

15. Zusammensetzung nach Anspruch 14, **dadurch gekennzeichnet, daß** die lipophile Phase ein Gemisch aus Glyzerinmono-, -di- und -triestern und PEG-Mono- und -Diestern mit der Gesamtheit der gesättigten Fettsäuren aus C₈-C₁₈ umfaßt, wobei das genannte Gemisch einen HLB-Wert gleich 14 aufweist und zwischen 50 und 95 Gewichtsprozent der Zusammensetzung darstellt.

16. Zusammensetzung nach Anspruch 14, **dadurch gekennzeichnet, daß** die Tensid-Phase zwischen 1 und 30 Gewichtsprozent des Gemisches darstellt.

17. Zusammensetzung nach Anspruch 14, **dadurch gekennzeichnet, daß** die Cotensid-Phase zwischen 3 und 32 Gewichtsprozent der Zusammensetzung darstellt.

18. Zusammensetzung nach Anspruch 14, **dadurch gekennzeichnet, daß** der Wirkstoff der Familie der Statine angehört.

19. Zusammensetzung nach Anspruch 18, **dadurch gekennzeichnet, daß** das Statin Simvastatin ist.

20. Zusammensetzung nach Anspruch 19, **dadurch gekennzeichnet, daß** das Simvastatin zwischen 0,1 und 6 Gewichtsprozent, vorteilhafterweise 4 Gewichtsprozent, der Zusammensetzung darstellt.

21. Zusammensetzung nach Anspruch 14, **dadurch gekennzeichnet, daß** sie folgende Gewichtsanteile umfaßt:
- zwischen 0,1 und 6% Simvastatin,
- zwischen 52 und 70% GELUCIRE® 44/14 (Lauryl-Macrogolglyzeride),
- zwischen 5 und 30% LABRASOL® (Capryl-Caprin-Macrogolglyzerid),
- zwischen 15 und 30% Propylenglykolmonocaprylat.

22. Zusammensetzung nach Anspruch 21, **dadurch gekennzeichnet, daß** das Propylenglykolmonocaprylat aus CAPRYOL® PGMC besteht, welches zwischen 15 und 25 Gewichtsprozent der Zusammensetzung darstellt.

23. Zusammensetzung nach Anspruch 21, **dadurch gekennzeichnet, daß** das Propylenglykolmonocaprylat aus CAPRYOL® 90 besteht und zwischen 20 und 30 Gewichtsprozent der Zusammensetzung darstellt.

24. Zusammensetzung nach Anspruch 14, **dadurch gekennzeichnet, daß** das Verhältnis TA/CoTA gleich 0,5 ist.
